# EUROPEAN PATENT SPECIFICATION

(11) **EP 2 790 667 B1**
(45) Date of publication and mention of the grant of the patent: **22.03.2017**
(21) Application number: 12801613.6
(22) Date of filing: 14.12.2012
(51) Int. Cl.: A61K 9/00, A61K 9/16, A61K 9/70

(54) **PROCESS FOR THE MANUFACTURING OF A DRUG DELIVERY SYSTEM BASED ON A POLYMER COMPRISING A DISPERSED BIOACTIVE AGENT**
VERFAHREN ZUR HERSTELLUNG EINES ARZNEIMITTELABGABESYSTEMS BASIEREND AUF EINEM POLYMER, UMFASSEND EINEN DISPERGIERTEN BIOAKTIVEN WIRKSTOFF
PROCÉDÉ POUR LA FABRICATION D'UN SYSTÈME D'ADMINISTRATION DE MÉDICAMENTS BASÉ SUR UN POLYMÈRE COMPRENANT UN AGENT BIOACTIF DISPERSÉ

(30) Priority: 16.12.2011 EP 11194043
(43) Date of publication of application: 22.10.2014
(73) Proprietor: DSM IP Assets B.V., 6411 TE Heerlen (NL)
(72) Inventor: ZUPANCICH, John Andrew, NL-6100 AA Echt (NL)
(74) Representative: Vandevijver, Pascale
(86) International application number: PCT/EP2012/075661
(87) International publication number: WO 2013/087903

(56) References cited:
- WO-A1-2009/012449
- WO-A1-2011/146483
- US-A1- 2007 134 332
- TSITLANADZE G ET AL: "Biodegradation of amino-acid-based poly(ester amide)s: in vitro weight loss and preliminary in vivo studies", JOURNAL OF BIOMATERIALS SCIENCE. POLYMER EDITION, VSP, UTRECHT, NL, vol. 15, 1 January 2004 (2004-01-01), pages 1-24, XP009129756, ISSN: 0920-5063, DOI: 10.1163/156856204322752200

## Description

The present invention relates to a process for the manufacturing of a drug delivery system based on a polymer comprising a dispersed bioactive agent. The present invention further relates to the drug delivery system obtainable by said process. The present invention further relates to the use of said drug delivery system in ophthalmology, cardiovascular, pain management, musculoskeletal, cancer treatment or in the delivery of vaccines.

An area of focus in the pharmaceutical industry is the development of drug delivery systems for controlled or sustained release of bioactive agents. Known drug delivery systems for sustained or controlled drug release are for example particles such as micro-and nanoparticles, fibres, rods, films or coatings. These drug delivery systems often comprise a drug dispersed in a biocompatible polymer matrix which can be implanted, administered orally or injected. The polymers most often used are for example poly-(lactic acid) or poly- (lactic acid-co-glycolic acid).

It is known that the manufacturing of a drug delivery system in which the bioactive agent is a protein causes difficult issues to deal with. It is for example a challenge to fabricate polymer/drug particles in which the drug is a protein. The size and morphology depends upon the fabrication method employed, and the formation of small polymer/drug particles in which the drug is a protein is currently limited to a few techniques. For example in Tabata et al., J. Cont. Release 23 55-64 (1993), it is disclosed that polymer/protein particles comprising poly(lactic acid) and either trypsin or insulin, were prepared by both an oil/water emulsion method and a neat mixing method at elevated temperature. The polymer/protein matrix thus formed was subsequently ground into particles. A disadvantage is that the particles prepared by the neat mixing method lost a significant fraction of protein activity, possibly due to the heating step. The particles also suffered from a large initial burst of protein release. The particles prepared by the oil/water emulsion method lost an even greater amount of protein activity, possibly caused by protein lability with respect to the oil. This means that the processes for the manufacturing of drug delivery systems comprising the incorporation of thermally sensitive or solvent sensitive bioactive agents or drugs, such as many proteins, peptides and polynucleotides is limited due to the process conditions which are often elevated temperatures (greater than about 45 C) and/or aqueous/organic emulsions. These process conditions result in a significant loss of drug activity. On the other hand it is known that processes utilizing a simple mixture of polymer with a solid bioactive agent or drug do often not yield a fine microscopic homogeneous dispersion of the bioactive agent or drug within the polymer matrix. This results in a more erratic drug release in vitro and in vivo.

The object of the present invention is to mitigate the above mentioned disadvantages. The object of the present invention is therefore to provide a new process for the manufacturing of drug delivery systems, based on a polymer comprising a bioactive agent or drug, suitable for injection or implantation, in which the drug may be a thermally or a solvent sensitive drug, while still maintaining high levels of activity and achieving a substantially homogeneous dispersion of the drug throughout the polymer.

Surprisingly it has been found that the above disadvantages can be overcome providing a process for the manufacturing of a drug delivery system based on a polymer comprising dispersed bioactive agent or drug via the following process steps:
(a) dissolving a polymer in an organic solvent
(b) mixing the dissolved polymer with the bioactive agent
(c) laminating the mixture between at least two sheets of polymeric material, whereby at least one sheet is permeable to the organic solvent,
(d) delaminating the two polymeric sheets to provide a film.

In WO-A-9742940 a process is disclosed for forming an implantable polymer/drug matrix mass. The method comprises the steps of (1) forming a polymer solution/drug mixture comprising a polymer dissolved in an organic solvent and a suspended drug such as a protein; (2) removing the solvent from the polymer solution/drug mixture, thereby forming a solid polymer/drug matrix; and (3) mechanically compressing the polymer/drug matrix, thereby forming an implantable polymer/drug matrix mass such as particles. In one embodiment, the polymer/drug matrix is formed by directing, for example, by pouring, atomizing, spraying or dripping, the polymer solution/drug mixture into a gas, which can be a liquefied gas, which is at a temperature sufficient to freeze the polymer solution/drug mixture, forming solid polymer solution/drug mixture particles. The solvent can then be removed from these particles by, for example, contacting the particles with a non-solvent at a temperature below the freezing point of the particles, whereby the solvent is extracted into the non-solvent, or by lyophilizing the particles in vacuum. In another embodiment, the polymer solution/drug mixture may be directed into a non-solvent, for example, ethanol or isopentane, at a temperature above the freezing point of the polymer/drug mixture, but still sufficiently low to cause precipitation of the polymer/drug matrix. This yields a non-solvent/polymer/drug matrix mixture, from which the polymer/drug matrix can be removed by filtration. A disadvantage of the above mentioned embodiments is that the organic solvent is still removed using process conditions which influence the stability and activity of sensitive drugs such as proteins in this case by the use of a second solvent called a non-solvent.

In the present invention the process conditions are such that the bioactive agent or drug is substantially homogeneously dispersed throughout the polymer matrix while still maintaining high levels of drug activity.

The polymer as referred to in step (a) can be any biostable, bioerodable or biodegradable polymer selected from the group consisting of polyesteramides, polythioesters, polyesterurethanes, polyesterurea's, PLLA, PLGA, poly(glycolic acid), poly(caprolactone), polyesters, polyanhydrides, polycarbonates, polyurethanes, polyurethane acrylate, poly (amino acids), polypeptides, polyamides, polysaccharides, polyethers, polysulfones, poly(meth)acrylates, polysiloxanes or polyolefins. Any suitable blends or copolymers of these materials can also be used.

The term biostable as used herein means a material not intended to degrade in vivo during the intended lifetime.

The terms biodegradable or bioerodable as used herein refers to material which is capable of being completely or substantially degraded or eroded when exposed to an in vivo environment or a representative in vitro. A polymer is capable of being degraded or eroded when it can be gradually broken-down, resorbed, absorbed and/or eliminated by, for example, hydrolysis, enzymolysis, oxidation, metabolic processes, bulk or surface erosion, and the like within a subject. It should be appreciated that traces or residue of polymer may remain following biodegradation.

Preferably the polymer is a biodegradable polymer selected from a polyesteramide comprising amino-acids. Still more preferably the polyesteramide has a chemical formula according to formula (I), 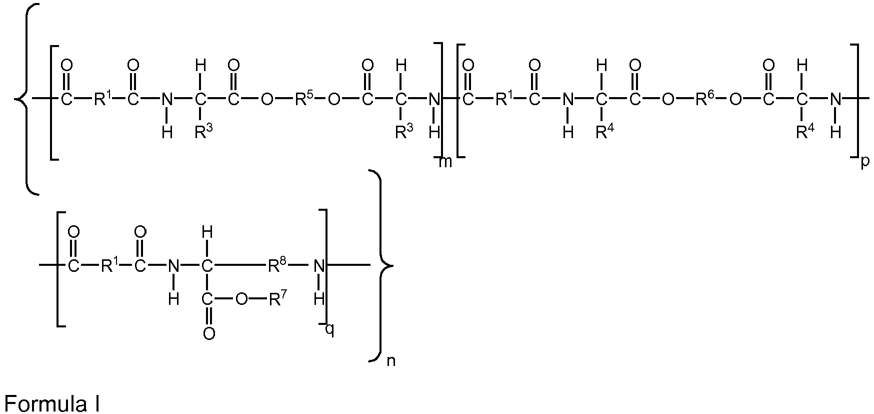 wherein
- m varies from 0.01 to 0.99; p varies from0.to 0.99; and q varies from 0.99 to 0.01; and wherein n is 5 to 100; and wherein
- R1 is independently selected from the group consisting of (C2-C20)alkylene, (C2-C20)alkenylene, -(R9-CO-O-R10-O-CO-R9)-, -CHR11-O-CO-R12-COOCR11- and combinations thereof;
- R3 and R4 in a single co-monomer m or p, respectively, are independently selected from the group consisting of hydrogen, (C1-C6)alkyl, (C2-C6)alkenyl, (C2-C6)alkynyl, (C6-C10)aryl, (C1-C6)alkyl, -(CH2)SH, -(CH2)2S(CH 3), -CH2OH, -CH(OH)CH3, -(CH2)4NH3+, --(CH2)3NHC(=NH2+)NH2, -CH2COOH, -(CH2)COOH, -CH2-CO-NH2, -CH2CH2-CO-NH2, -CH2CH2COOH, CH3-CH2-CH(CH3)-, (CH3)2-CH-CH2-, H2N-(CH2)4-, Ph-CH2-, CH=C-CH2-, HO-p-Ph-CH2-, (CH3)2-CH-, Ph-NH-, NH-(CH2)3-C-, NH-CH=N-CH=C-CH2-.
- R5 is selected from the group consisting of (C2-C20)alkylene, (C2-C20)alkenylene, alkyloxy or oligoethyleneglycol
- R6 is selected from bicyclic-fragments of 1,4:3,6-dianhydrohexitols of structural formula (II); 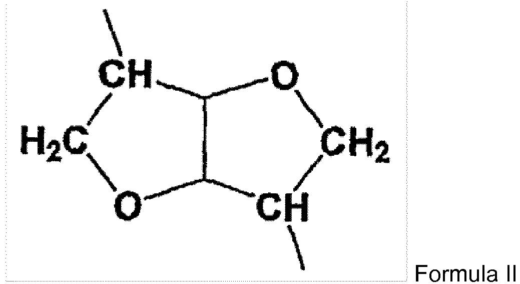
- R7 is hydrogen, (C6-C10) aryl, (C1-C6) alkyl or a protecting group such as benzyl- or a bioactive agent;
- R8 is independently (C1-C20) alkyl or (C2-C20)alkenyl;
- R9 or R10 are independently selected from C2-C12 alkylene or C2-C12 alkenylene.
- R11 or R12 are independently selected from H, methyl, C2-C12 alkylene or C2-C12 alkenylene.

In a further embodiment of the present invention the polyesteramide may also comprise a structure according to formula (III) 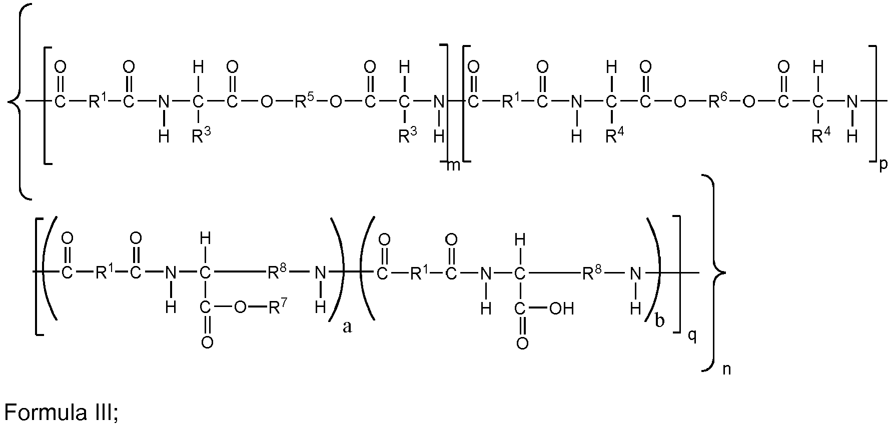 wherein
- m+p varies from 0.9-0.1 and q varies from 0.1 to 0.9
- m+p+q=1 whereby m or p could be 0
   a is at least 0.05, b is at least 0.05 whereby a+b=1
- n is 5 to 300;
- R₁ is independently selected from the group consisting of (C₂-C₂₀) alkylene, (C₂-C₂₀) alkenylene, -(R₉-CO-O-R₁₀-O-CO-R₉)-, -CHR₁₁-O-CO-R₁₂-COOCR₁₁- and combinations thereof;
- R₃ and R₄ in a single backbone unit m or p, respectively, are independently selected from the group consisting of hydrogen, (C₁-C₆)alkyl, (C₂-C₆)alkenyl, (C₂-C₆)alkynyl, (C₆-C₁₀)aryl, (C₁-C₆)alkyl, -(CH₂)SH, -(CH₂)₂S(CH₃), -CH₂OH, -CH(OH)CH₃, -(CH₂)₄NH₃+, -(CH₂)₃NHC(=NH₂+)NH₂, -CH₂COOH, -(CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂-.
- R₅ is selected from the group consisting of (C₂-C₂₀)alkylene, (C₂-C₂₀)alkenylene, alkyloxy or oligoethyleneglycol
- R₆ is selected from bicyclic-fragments of 1,4:3,6-dianhydrohexitols of structural formula (II)
- R₇ is selected from the group consisting of (C₆-C₁₀) aryl (C₁-C₆) alkyl
- R₈ is -(CH₂)₄-;
- R₉ or R₁₀ are independently selected from C₂-C₁₂ alkylene or C₂-C₁₂ alkenylene.
- R₁₁ or R₁₂ are independently selected from H, methyl, C₂-C₁₂ alkylene or C₂-C₁₂ alkenylene

In one embodiment the biodegradable polyesteramide copolymer according to Formula (III) comprises p=0 and m+q=1 whereby m=0.75, a=0.5 and a+b=1 R₁ is (CH₂)₈, R₃ is (CH₃)₂-CH-CH₂-, R₅ is hexyl, R₇ is benzyl and R₈ is -(CH2)4-.

In another embodiment of the present invention the biodegradable polyesteramide copolymer according to Formula (III) comprises m+p+q=1, q=0.25, p=0.45 and m=0.3 whereby a is 0.5 and a+b=1 and whereby R₁-(CH₂)₄; R₃ and R₄ respectively are (CH₃)₂-CH-CH₂-, R₅ is selected from the group consisting of (C₂-C₂₀)alkylene, R₆ is selected from bicyclic-fragments of 1,4:3,6-dianhydrohexitols of structural formula (II), R₇ is benzyl and R₈ is -(CH2)4-.

In a still further embodiment of the present invention the biodegradable polyesteramide copolymer according to Formula (III) comprises m=0, p+q=1 and p=0.75 whereby a= 0.5 and a+b=1, R₁ is-(CH₂)₄; R₄ is (CH₃)₂-CH-CH₂-, R₇ is benzyl, R₈ is -(CH₂)₄- and R₆ is selected from bicyclic fragments of 1,4:3,6-dianhydrohexitols of structural formula (II).

In another further embodiment of the present invention the biodegradable poly(esteramide) copolymer according to Formula (III) comprises m+p+q=1, q=0.1, p=0.30 and m=0.6 whereby a=0.5 and a+b=1. R₁-(CH₂)₄; R₃ and R₄ respectively, are (CH₃)₂-CH-CH₂-; R₅ is selected from the group consisting of (C₂-C₂₀)alkylene, R₇ is benzyl, R₈ is -(CH₂)₄- and R₆ is selected from bicyclic-fragments of 1,4:3,6-dianhydrohexitols of structural formula (II);

As used herein, the term "alkyl" refers to a straight or branched chain hydrocarbon group including methyl, ethyl, n-propyl, isopropyl, n-butyl, isobutyl, tert-butyl, n-hexyl, and the like.

As used herein, the term "alkylene" refers to a divalent branched or unbranched hydrocarbon chain containing at least one unsaturated bond in the main chain or in a side chain.

As used herein, the term "alkenyl" refers to a straight or branched chain hydrocarbon group containing at least one unsaturated bond in the main chain or in a side chain.

As used herein, "alkenyl" or "alkenylene", refers to structural formulas herein to mean a divalent branched or unbranched hydrocarbon chain containing at least one unsaturated bond in the main chain or in a side chain.

As used herein, "alkynyl", refers to straight or branched chain hydrocarbon groups having at least one carbon-carbon triple bond.

The term "aryl" is used with reference to structural formulas herein to denote a phenyl radical or an ortho-fused bicyclic carbocyclic radical having about nine to ten ring atoms in which at least one ring is aromatic. Examples of aryl include, but are not limited to, phenyl, naphthyl, and nitrophenyl.

The polyesteramide copolymers preferably have an average number molecular weight (Mn) ranging from 15,000 to 200,000 Daltons. The polyesteramide copolymers described herein can be fabricated in a variety of molecular weights and a variety of relative proportions of the m, p, and q units in the backbone. The appropriate molecular weight for a particular use can be readily determined by one of skill in the art. A suitable Mn will be in the order of about 15,000 to about 100,000 Daltons, for example from about 30,000 to about 80,000 or from about 35,000 to about 75,000. Mn is measured via GPC in THF with polystyrene as standard.

The basic polymerization process of polyesteramides is based on the process described by G. Tsitlanadze, et al. J. Biomater. Sci. Polym. Edn. (2004) 15:1-24, however different building blocks and activating groups were used.

The polyesteramides of the present invention are for example synthesized as shown in scheme 1; via solution polycondensation of para-toluene sulfonate di-amines salts (X1, X2, X3) with activated di-acids (Y1). Typically dimethylsulfoxide or dimethylformamide are used as solvent. Typically as a base triethylamide is added, the reaction is carried out under an inert atmosphere at 60°C for 24-72hours under constant stirring. Subsequently the obtained reaction mixture is purified via a water precipitation followed by an organic precipitation and filtration. Drying under reduced pressure yields the polyesteramide.

The organic solvent as referred to in step (a) may be selected from methylene chloride, acetone, ethyl acetate, methyl acetate, tetrahydrofuran, ethanol, methanol, isopropanol and chloroform. The organic solvent used depends on the polymer to be dissolved and on the polymer sheet used in process step (c) because the organic solvent should be permeable to the polymer sheet. The choice can however be readily determined by one of skill in the art. In the present invention methylene chloride, ethanol or methanol are preferably used as the organic solvent.

In step (b) of the process of the present invention the dissolved polymer is mixed with a bioactive agent or drug by agitation, for example by shaking, stirring, vortexing, homogenizing or sonicating. The bioactive agent can be present, for example, as a powder, which can be crystalline, semi-crystalline or amorphous. The bioactive agent can also be present as a liquid. If present as a powder the bioactive agent is suspended in the polymer solution, if present in the liquid form the bioactive agent is either co-dissolved, emulsified or dispersed in the polymer solution.

The weight of bioactive agent or drug relative to the weight of dissolved polymer can range from about 0.02-100 wt%. Preferably it can range from 1-75 wt%, more preferably it can range from 10-50 wt%.

The bioactive agent can be any agent which is a therapeutic, prophylactic, or diagnostic agent. Such bioactive agent may include without any limitation small molecule drugs or biologics. Examples of biologics are proteins such as immunoglobulin-like proteins, antibodies, cytokines (e.g., lymphokines, monokines, chemokines), interleukins, interferons, erythropoietin (also referred to herein as "EPO"), nucleases, tumor necrosis factor, colony stimulating factors, insulin, enzymes, tumor suppressors, hormones (e.g., growth hormone and adrenocorticotrophic hormone), antigens (e.g., bacterial and viral antigens), growth factors, polypeptides and polynucleotides, such as antisense molecules. Examples of small molecule drugs can have antiproliferative or anti-inflammatory properties or can have other properties such as antineoplastic, antiplatelet, anti-coagulant, anti-fibrin, antithrombotic, antimitotic, antibiotic, antiallergic, or antioxidant properties. Examples of antiproliferative agents include rapamycin and its functional or structural derivatives, 40-O-(2-hydroxy)ethyl-rapamycin (everolimus), and its functional or structural derivatives, paclitaxel and its functional and structural derivatives. Examples of rapamycin derivatives include ABT-578, 40-0-(3-hydroxy)propyl-rapamycin, 40-O-[2-(2-hydroxy)ethoxy]ethyl-rapamycin, and 40-0-tetrazole-rapamycin. Examples of paclitaxel derivatives include docetaxel. Examples of antineoplastics and/or antimitotics include methotrexate, azathioprine, vincristine, vinblastine, fluorouracil, doxorubicin hydrochloride (e.g. Adriamycin(R) from Pharmacia AND Upjohn, Peapack NJ.), and mitomycin (e.g. Mutamycin(R) from Bristol-Myers Squibb Co., Stamford, Conn.). Examples of such antiplatelets, anticoagulants, antifibrin, and antithrombins include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein Hb/nia platelet membrane receptor antagonist antibody, recombinant hirudin, thrombin inhibitors such as Angiomax (Biogen, Inc., Cambridge, Mass.), calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor(R) from Merck AND Co., Inc., Whitehouse Station, NJ), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), super oxide dismutases, super oxide dismutase mimetic, 4-amino-2,2,6,6-tetramethylpiperidine-1-oxyl (4-amino-TEMPO), estradiol, anticancer agents, dietary supplements such as various vitamins, and a combination thereof. Examples of anti-inflammatory agents including steroidal and nonsteroidal anti-inflammatory agents include biolimus, tacrolimus, dexamethasone, clobetasol, corticosteroids or combinations thereof. Examples of such cytostatic substances include angiopeptin, angiotensin converting enzyme inhibitors such as captopril (e.g. Capoten(R) and Capozide(R) from Bristol-Myers Squibb Co., Stamford, Conn.), cilazapril or lisinopril (e.g. Prinivil(R) and Prinzide(R) from Merck AND Co., Inc., Whitehouse Station, NJ). An example of an antiallergic agent is permirolast potassium. Other therapeutic substances or agents which may be appropriate include alpha-interferon, pimecrolimus, imatinib mesylate, midostaurin, and genetically engineered epithelial cells. The foregoing substances can also be used in the form of prodrugs or co-drugs thereof. The foregoing substances also include metabolites thereof and/or prodrugs of the metabolites. The foregoing substances are listed by way of example and are not meant to be limiting.

The polymer /drug mixture may further comprise one or more excipients, including saccharides and their derivatives (e.g., disaccharides, polysaccharides, sugar alcohols), proteins (e.g., gelatin), synthetic polymers (e.g., polyvinylpyrrolidone, polyethylene glycol), antioxidants (e.g., vitamin A, vitamin E, vitamin C, retinyl palmitate), amino acids (i.e., glycine, cysteine, histidine, methionine), acids (e.g., citric acid), salts (e.g., sodium chloride), including bases, surfactants, stabilizers and/or a release modifying agent. The polymer /drug mixture may further comprise one or more additional drugs.

In process step (c) the resulting mixture of process step (b) is laminated between at least two sheets of polymeric material, whereby at least one of the sheets is permeable to the organic solvent.

The sheets of polymeric material for example comprise a polymeric elastomer material selected from the group consisting of silicon rubber, ethylene-propylene elastomers, polyurethanes or crosslinked polymers thereof. Preferably the sheets are flexible. The sheets are preferably made from silicone rubber selected from the group consisting of a medical grade and/or food contact grade silicone rubber, for example PDMS.

After the laminating step (c) the two polymeric sheets are delaminated in step (d). The delamination step comprises for example separating the two polymeric sheets from drug-loaded film via peeling and/or scraping. It is also possible to remove a single polymeric sheet, contacting the partially exposed drug-loaded film with another substrate and transferring the drug-loaded film completely to the other substrate via removal of the second polymer sheet via peeling and/or scraping. After delamination, a drug loaded film is provided which can be further processed into a subsequently implantable or injectable drug delivery system such as a fiber, rod, disc, coating, tube or rolled film. The film can therefore be shaped into other geometries and/or cut into smaller pieces. The film preferably has a thickness in the range of 1 micrometer to 1 mm.

It is of course possible to further stretch the film to a desired thickness in the nanometer range.

In a further embodiment of the present invention the drug delivery system may comprise a barrier layer to improve drug release characteristics. The barrier layer may comprise a polymer selected from the group of polyesteramides, polythioesters, polyesterurethanes, polyesterurea's, PLLA, PLGA, poly(glycolic acid), poly(caprolactone), polyesters, polyanhydrides, polycarbonates, polyurethanes, polyurethane acrylate, poly (amino acids), polypeptides, polyamides, polysaccharides, polyethers, polysulfones, poly(meth)acrylates, polysiloxanes or polyolefins. The barrier layer can be applied by dip coating or spray coating.

The process steps (a), (b) and (c) according to the present invention thus produce a substantial homogeneous dispersion of the drug throughout the polymer without using heat extrusion. The process of the present invention further allows the use of process conditions such as a low temperature and a mild solvent removal. Therefore this process is in particular suitable for thermally or solvent sensitive or "labile" drugs such as many proteins, polypeptides or polynucleotides.

The term "labile" drug as used herein refers to a drug which loses a substantial amount of activity when either warmed to elevated temperatures, such as temperatures greater than physiological temperatures (about 37 C), or dissolved in an organic solvent or in solution at an aqueous/organic interface. The process of the present invention thus allows the formation of drug delivery systems maintaining a high degree of the drug, for example, protein activity present prior to processing.

The term "polymer comprising dispersed bioactive agent or drug", as used herein, refers to a solid material comprising a polymer, copolymer or polymer blend, and drug molecules, which are dispersed throughout the polymer. The polymer comprising dispersed drug may further comprise excipients, such as surfactants or sugars, release modifying agents, such as metal-containing salts, or one or more additional drug substances.

The invention will now be further and specifically described by the following examples.

### FIGURES

Figure 1. Example aqueous SEC-HPLC chromatogram A) release sample containing rHGH; B) release medium: rHGH elutes at approximately 17 min.
Figure 2. Bioactivity assay results. Concentration of rHGH in the cell culture medium was determined by SEC-HPLC (light bars). Concentration of active rHGH in the cell culture medium as determined by the cell assay (dark bars). "A" denotes release samples that have been autoclaved prior to introduction to the cell culture medium (negative control). Samples denoted with an asterisk (*): calculated concentrations of "active" rHGH exceed 250 pg/mL.

### MATERIALS

-In example 1a polymer solution comprising 10 wt% solution of PEA-III-Ac-Bz in dichloromethane was used. PEA-III-Ac-Bz means a PEA co-polymer of formula I wherein m is about 0.3, p is about 0.45, q is about 0.25, n is about 50 and
- R₁ is (CH₂)₈;
- R₃ and R₄ are selected from (CH₃)₂-CH-CH₂- ;
- R₅ is selected from (CH₂)_{6;}
- R₆ is 1,4 :3,6-dianhydrosorbitol (DAS);
- R₇ is a benzyl protecting group;
- R₈ is (CH₂)_{4.}

-Further a solid formulation of lyophilized powder of recombinant human growth hormone (rHGH) was used as the bioactive agent. As polymer sheets, silicone rubber sheets (PDMS), food contact grade following FDA code CFR21 were used having a smooth surface and 1 mm thickness, approx. 10x15 cm in size. These are available from Profiles Market, BP 80006 - 28631 Gellainville.

A PDMS mold was used based on a food contact grade with a 2 mm thickness, and approx. 5x7 cm in size having a 20 mm diameter circular hole in the center.

In example 2, a polymer solution was used comprising 50 wt% of PEAIII-Ac-Bz in absolute ethanol. As the bioactive agent an aqueous solution containing fluorescein-labeled bovine serum albumin (FITC-BSA), trehalose and polyethylene glycol (PEG) was used. The PDMS sheets with a thickness of approx. 2 mm, 7x7 cm were synthesized in-house based on a research grade from Sylgard 184 silicone elastomer kit available from Dow Corning.

In example 5, a polymer solution was used comprising 6wt% of PEA-III-Ac-Bz in dichloromethane. As the bioactive agent a lyophilized powder of recombinant human growth hormone (rhGH; Genotropin Miniquick 2mg; Pfizer; Kent, NJ U.S.A) was used. The PDMS sheets are smooth, made of a food contact grade and have a thickness of 1 mm 10x15 cm. The Teflon sheet comprises a PTFE coating and has a thickness of 70 micrometer.

### Example 1: Preparation of a solid dispersion comprising PEA-III-Ac-Bz and rHGH

A PDMS mold was placed in the center of a PDMS sheet. rHGH powder (∼25 mg) was dosed into the mold, followed by the addition of the polymer solution (∼125 mg). The two components were mixed by hand with a spatula until a white, opaque paste was obtained. The mixture was allowed to sit at room temperature for five minutes prior to removal of the mold. A second PDMS sheet was placed on top of the first and a film was pressed by passing this stack multiple times through a hand operated roll press. Vacuum was then applied to the stack to quickly remove remaining solvent. After two hours under vacuum, the PDMS sheets could easily be pealed off leaving a solid protein-loaded polymer film. Vacuum was again applied to the polymer film at room temperature to remove all residual solvent.

The film was subsequently cut into fibers and the rHGH present in the fibers was released in a release medium. The bioactivity of rHGH was retained as determined by a cell proliferation assay.

### Example 2: Preparation of a liquid dispersion of PEA-III-Ac-Bz and fluorescein-labeled bovine serum albumin (FITC-BSA)

A polymer solution (∼100 mg) was placed in center of a PDMS sheet and liquid API solution (∼20 mg) was added. The two components were then mixed by hand with a spatula until visually homogenous. The mixture was then placed under vacuum at (∼200 mbar) to degas. A second PDMS sheet was placed on top of the first and a film was pressed by applying even pressure through use of a -1.5 kg mass. After drying overnight at room temperature, the protein-loaded film was easily removed from the PDMS sheets.

Fibers where then cut from the film and homogeneity of the protein dispersion was confirmed with bright field and fluorescence microscopy. Minimal release of FITC-BSA was observed when fibers were exposed to buffer confirming microscopy evidence of good dispersion of protein rich domains within the polymer matrix.

### Example 3: Release and bioactivity experimental details

PEA-III-Ac-Bz fibers in which approximately 1-2wt% rHGH (Creative BioMart; Shirley, NY U.S.A) was contained within the polymer matrix, prepared as described in example 1, were exposed to predetermined volumes of release medium at 37 °C over set periods of time. The release medium was composed of phosphate buffered saline (pH 7.4) with added bovine serum albumin and penicillin. After a set period time, during which fibers were submerged in the release medium and incubated at 37 °C, the total volume of release medium was removed with a mechanical pipette and stored at 4 °C for subsequent analysis. A known volume of fresh release medium was then added to the fibers with a mechanical pipette and incubation at 37 °C continued. After set periods of time, the release medium was removed and replaced in the same manner as stated above.

The concentration of rHGH present in release samples was determined through use of aqueous size exclusion - high performance liquid chromatography (SEC-HPLC).

The method used is described below:
- Analysis of release samples were carried out on an Agilent 1200 Series system equipped with a TSKgel G2000SWXL 7.8*300 mm (TOSOH Bioscience) column, Col No 2SWX02SS4835
- Mobile phase: 1.059 mM KH₂PO₄, 2.966 mM Na₂HPO4, 300 mM NaCl, pH=7.4, 10% EtOH (287.16 mg KH₂PO₄, 841.1 mg Na₂HPO₄, 35.64 g NaCl in 2L Milli-Q water, pH adjusted at 7.4 with NaOH 1 N, 222 mL EtOH)
- Conditions: Flow 0.5 mL/min for 35 minutes, detection at 220, 250 and 280 nm.
- Response factor was calculated from a reference rHGH sample (Creative BioMart). rHGH content of this reference was determined by Bradford assay. Response factor was used to calculate the concentration of rHGH in release samples.
- Bioactivity of rHGH present in release samples was assessed by measuring its influence on the proliferation of Nb2 (rat lymphoma) cells.¹

The method used is described below:²
- Nb2 cells (Sigma-Aldrich) derived from rat T lymphoma cells were cultured in suspension in Fischer's medium supplemented with 10% fetal bovine serum, 10% horse serum, 50 µM 2-mercaptoethanol and 2% penicillin/streptomycin ("culture medium") in a humidified incubator at 37°C (5% CO₂). For proliferation assays, cells growing at log- phase were washed two times with the same medium prepared without fetal bovine serum ("incubation medium") and kept for 24 hours in this medium.
- Cells were then counted with a Guava Easycyte (Millipore) capillary cytometer using Viacount reagent (Millipore) to stain cells, according to the recommendations of the manufacturer. The cells suspension was diluted in incubation medium to reach 200.000 viable cells per mL. Cells were plated in 96-well plates (100 µL cell suspension per well).
- Samples originating from the release experiments were diluted in incubation medium to reach an expected (according to HPLC quantification) concentration of rHGH between 80 and 280 pg/mL(concentration range in which growth of Nb2 cells is rHGH concentration-dependent).
- These solutions were split in two aliquots and one aliquot was autoclaved. 100 µL of these solutions were added to the Nb2 cells, and cells were incubated for 72 hours at 37°C.
- After incubation, cells were stained with 50uL Viacount reagent and viable cells were counted by capillary cytometry.

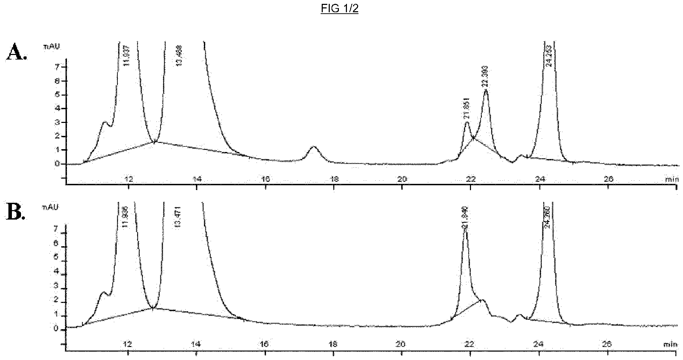

The concentration of rHGH present in release samples at time points from 1 hour to 48 hours was measured using aqueous SEC-HPLC by correlation of peak area (i.e., 17 min elution volume) to rHGH concentration through use of a calibration curve (Figure 1).

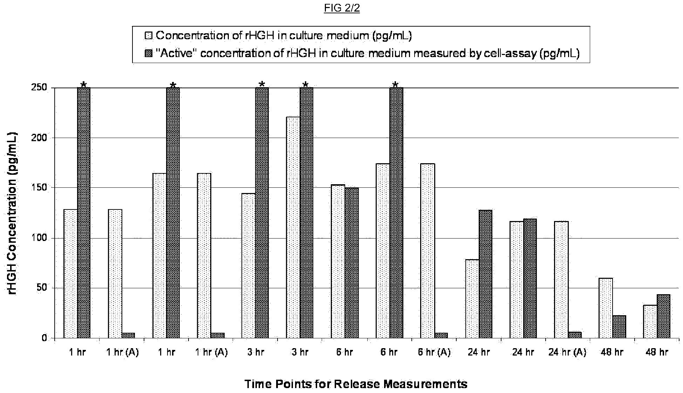

Bioactivity of rHGH released from the fibers was measured through use of the cell-proliferation assay described above. Release samples were introduced to the cell culture medium and the effect of rHGH on cell proliferation was measured via cell counting with capillary cytometry. A positive cell response was measured from release samples taken at 1 h, 3h, 6h, 24h & 48h, indicating that rHGH released from the fibers was bioactive (Figure 2).
1 Bozzola, M. et. al. Evaluation of growth hormone bioactivity using the Nb2 cell bioassay in children with growth disorders. J. Endocrinol Invest.1998, 21, 765-770
2 Tanaka. T. et. al. A new sensitive and specific bioassay for lactogenic hormones: measurement of prolactin and growth hormone in human serum. J. Clin. Endocrinol. Metab.1980, 51, 1058-1063

### Example 4

As a control, release samples were taken (from the fibers processed according to the present invention). At 1 h, 6h, and 24h the fibers were exposed to elevated temperature and pressure (i.e.autoclave) in order to denature and/or deactivate the rHGH. A qualitative difference in cell response to release samples before and after autoclaving was recorded, validating our experimental method. These results confirm that rHGH present in and subsequently released from the fibers retains its bioactivity even under the harsh conditions.

### Example 5

rhGH containing powder (3.7 mg solid containing 2 mg or rhGH) was dosed onto a Teflon sheet, followed by the addition of the polymer solution (PEA-III-Ac-Bz in dichloromethane (∼250 mg). The two components were blended by hand with a spatula until a white, opaque paste was obtained. A PDMS sheet was placed on top of the blended material, the Teflon sheet removed, and a second PDMS sheet was placed on the first. This stack was then pressed by multiple passages through a hand operated roll press. The film was allowed to sit at room temperature for five minutes after which the PDMS sheets were easily peeled away leaving a solid protein-loaded polymer film.

## Claims

1. Process for the manufacturing of a drug delivery system based on a polymer comprising dispersed bioactive agent via the following process steps:
(a) dissolving a polymer in an organic solvent
(b) mixing the dissolved polymer with the bioactive agent
(c) laminating the mixture between at least two sheets of polymeric material, whereby at least one sheet is permeable to the organic solvent,
(d) delamination of the two polymeric sheets to provide a film.

2. Process according to claim 1 wherein the film has a thickness in the range of 1 micrometer to 1 mm.

3. Process according to any one of the claims 1-2 in which the film is processed into a drug delivery system suitable for implantation or injection.

4. Process according to any one of the claims 1-3 whereby the drug delivery system comprises fibres, rods, discs, coatings, tubes, films or rolled films.

5. Process according to claim 1 whereby the polymer is a biostable, bioerodable or biodegradable polymer.

6. Process according to claim 5 whereby the biostable, bioerodable or biodegradable polymer may be selected from the group consisting of polyesteramides, polythioesters, polyesterurethanes, polyesterurea's, PLLA, PLGA, polyesters, polyanhydrides, polycarbonates, polyurethanes, poly (amino acids), polypeptides, polyamides, polysaccharides, polyethers, polysulfones, poly(meth)acrylates, polysiloxanes, polyolefins.

7. Process according to claim 6 whereby the biodegradable polymer is selected from a polyesteramide comprising amino-acids.

8. Process according to any one of the claims 6-7 whereby the polyesteramide (PEA) has a chemical formula according to formula (I), 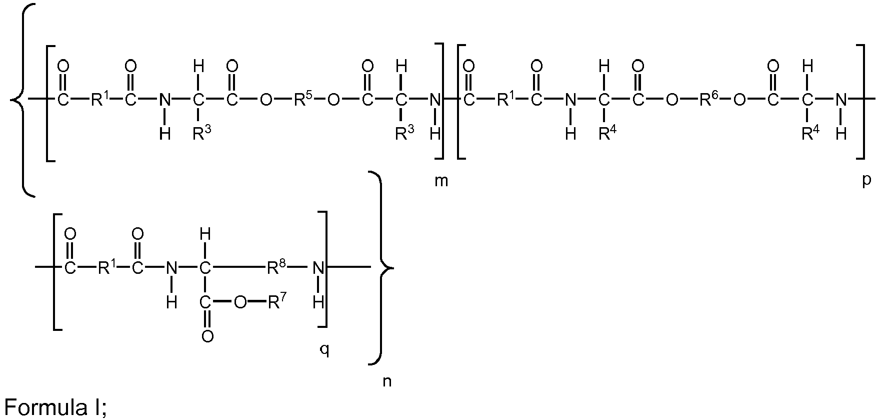 wherein
- m varies from 0.01 to 0.99; p varies from 0 to 0.99; and q ivaries from 0.99 to 0.01; and wherein n is 5 to 100; and wherein
- R₁ is independently selected from the group consisting of (C₂-C₂₀)alkylene, (C₂-C₂₀)alkenylene, -(R₉-CO-O-R₁₀-O-CO-R₉)-, -CHR₁₁-O-CO-R₁₂-COOCR₁₁- and combinations thereof;
- R₃ and R₄ in a single co-monomer m or p, respectively, are independently selected from the group consisting of hydrogen, (C₁-C₆)alkyl, (C₂-C₆)alkenyl, (C₂-C₆)alkynyl, (C₆-C₁₀)aryl, (C₁-C₆)alkyl, -(CH₂)SH, -(CH₂)₂S(CH₃), -CH₂OH, -CH(OH)CH₃, -(CH₂)₄NH₃+, --(CH₂)₃NHC(=NH₂+)NH₂, -CH₂COOH, -(CH₂)COOH, -CH₂-CO-NH₂, -CH₂CH₂-CO-NH₂, -- -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂-.
- R₅ is selected from the group consisting of (C₂-C₂₀)alkylene, (C₂-C₂₀)alkenylene, alkyloxy or oligoethyleneglycol
- R₆ is selected from bicyclic-fragments of 1,4:3,6-dianhydrohexitols of structural formula (II); 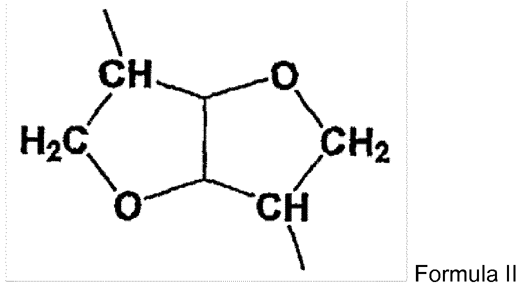
- R₇ is hydrogen, (C₆-C₁₀) aryl, (C₁-C₆) alkyl or a protecting group such as benzyl- or a bioactive agent;
- R₈ is independently (C₁-C₂₀) alkyl or (C₂-C₂₀)alkenyl;
- R₉ or R₁₀ are independently selected from C₂-C₁₂ alkylene or C₂-C₁₂ alkenylene.
- R₁₁ or R₁₂ are independently selected from H, methyl, C₂-C₁₂ alkylene or C₂-C₁₂ alkenylene.

9. Process according to any one of the claims 6-7 whereby the polyesteramide (PEA) has a chemical formula according to formula (III), 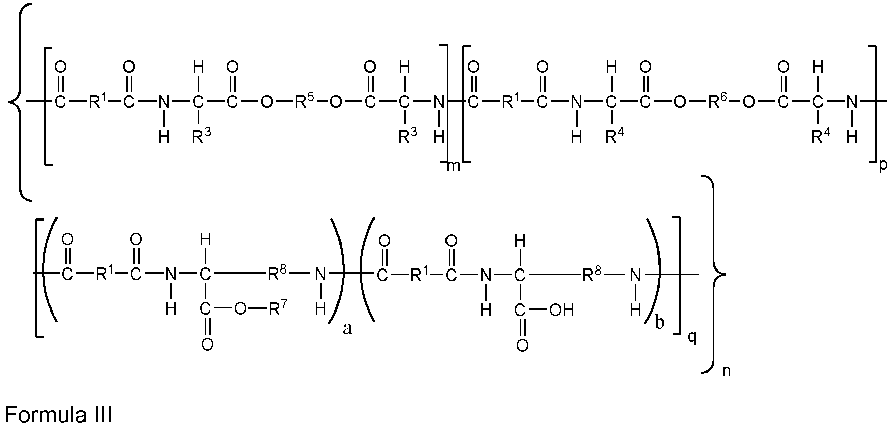 wherein
- m+p varies from 0.9-0.1 and q varies from 0.1 to 0.9
- m+p+q=1 whereby m or p could be 0 a is at least 0.05, b is at least 0.05 whereby a+b=1
- n is 5 to 300;
- R₁ is independently selected from the group consisting of (C₂-C₂₀) alkylene, (C₂-C₂₀) alkenylene, -(R₉-CO-O-R₁₀-O-CO-R₉)-, -CHR₁₁-O-CO-R₁₂-COOCR₁₁- and combinations thereof;
- R₃ and R₄ in a single backbone unit m or p, respectively, are independently selected from the group consisting of hydrogen, (C₁-C₆)alkyl, (C₂-C₆)alkenyl, (C₂-C₆)alkynyl, (C₆-C₁₀)aryl, (C₁-C₆)alkyl, -(CH₂)SH, -(CH₂)₂S(CH₃), -CH₂OH, -CH(OH)CH₃, -(CH₂)₄NH₃+, -(CH₂)₃NHC(=NH₂+)NH₂, -CH₂COOH, -(CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂-.
- R₅ is selected from the group consisting of (C₂-C₂₀)alkylene, (C₂-C₂₀)alkenylene, alkyloxy or oligoethyleneglycol
- R₆ is selected from bicyclic-fragments of 1,4:3,6-dianhydrohexitols of structural formula (II)
- R₇ is selected from the group consisting of (C₆-C₁₀) aryl (C₁-C₆) alkyl
- R₈ is -(CH₂)₄-;
- R₉ or R₁₀ are independently selected from C₂-C₁₂ alkylene or C₂-C₁₂ alkenylene.
- R₁₁ or R₁₂ are independently selected from H, methyl, C₂-C₁₂ alkylene or C₂-C₁₂ alkenylene

10. Process according to any one of the claims 1-9 whereby the bioactive agent is selected from the group of small molecule drugs or biologics.

11. Process according to claim 10 whereby the biologic is selected from the group of proteins, peptides or polynucleotides.

12. Process according to claim 1 whereby at least one of the sheets comprises an elastomeric material.

13. Process according to claim 12 whereby the elastomeric material is selected from the group consisting of silicon rubber, ethylene-propylene elastomers, or polyurethanes.

14. Process according to claim 13 whereby the silicon rubber is selected from the group consisting of a medical grade and/or food contact grade silicone rubber.

15. Drug delivery system obtainable by the process according to any one of the claims 1-14.

16. Drug delivery system according to claim 15 for use in ophthalmology, cardiovascular, pain management, musculoskeletal, cancer treatment or in vaccine delivery.

## Patentansprüche

1. Verfahren zur Herstellung eines auf einem einen dispergierten bioaktiven Stoff umfassenden Polymer basierenden Arzneistoffzuführungssystems über die folgenden Verfahrensschritte:
(a) Lösen eines Polymers in einem organischen Lösungsmittel,
(b) Mischen des gelösten Polymers mit dem bioaktiven Stoff,
(c) Laminieren des Gemischs zwischen wenigstens zwei Schichten Polymermaterial, wobei wenigstens eine Schicht für das organische Lösungsmittel durchlässig ist,
(d) Delamination der beiden Polymerschichten, so dass eine Folie bereitgestellt wird.

2. Verfahren nach Anspruch 1, wobei die Folie eine Dicke im Bereich von 1 Mikrometer bis 1 mm aufweist.

3. Verfahren nach einem der Ansprüche 1-2, bei dem die Folie zu einem für Implantation oder Injektion geeigneten Arzneistoffzuführungssystem verarbeitet wird.

4. Verfahren nach einem der Ansprüche 1-3, wobei das Arzneistoffzuführungssystem Fasern, Stäbchen, Scheiben, Beschichtungen, Röhrchen, Folien oder gerollte Folien umfasst.

5. Verfahren nach Anspruch 1, wobei es sich bei dem Polymer um ein biologisch stabiles, biologisch erodierbares oder biologisch abbaubares Polymer handelt.

6. Verfahren nach Anspruch 5, wobei das biologisch stabile, biologisch erodierbare oder biologisch abbaubare Polymer aus der aus Polyesteramiden, Polythioestern, Polyesterurethanen, Polyesterharnstoffen, PLLA, PLGA, Polyestern, Polyanhydriden, Polycarbonaten, Polyurethanen, Polyaminosäuren, Polypeptiden, Polyamiden, Polysacchariden, Polyethern, Polysulfonen, Poly(meth)acrylaten, Polysiloxanen, Polyolefinen bestehenden Gruppe ausgewählt ist.

7. Verfahren nach Anspruch 6, wobei das biologisch abbaubare Polymer aus einem Aminosäuren umfassenden Polyesteramid ausgewählt ist.

8. Verfahren nach einem der Ansprüche 6-7, wobei das Polyesteramid (PEA) eine chemische Formel gemäß der Formel (I) aufweist, 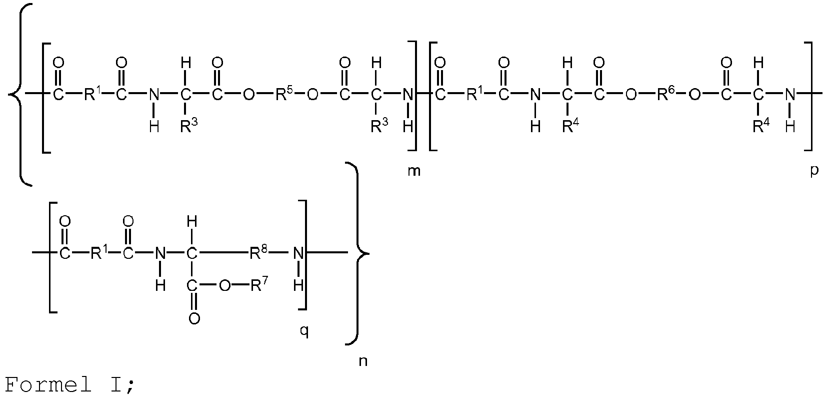 wobei
- m von 0,01 bis 0,99 variiert; p von 0 bis 0,99 variiert; und q von 0,99 bis 0,01 variiert; und wobei n gleich 5 bis 100 ist; und wobei
- R₁ unabhängig aus der aus (C₂-C₂₀)Alkylen, (C₂-C₂₀)-Alkenylen, - (R₉-CO-O-R₁₀-O-CO-R₉)-, -CHR₁₁-O-CO-R₁₂-COOCR₁₁- und Kombinationen davon bestehenden Gruppe ausgewählt ist;
- R₃ und R₄ in einem einzelnen Comonomer m bzw. p unabhängig aus der aus Wasserstoff, (C₁-C₆)Alkyl, (C₂-C₆)Alkenyl, (C₂-C₆)Alkinyl, (C₆-C₁₀)Aryl, (C₁-C₆)Alkyl, -(CH₂)SH, - (CH₂)₂S(CH₃), -CH₂OH, -CH(OH)CH₃, -(CH₂)₄NH₃+, -(CH₂)₃NHC(=NH₂+)NH₂, -CH₂COOH, -(CH₂)COOH, -CH₂-CO-NH₂, -CH₂CH₂-CO-NH₂, - -CH₂CH₂COOH, CH₃-CH₂-CH(CH₃)-, (CH3) 2-CH-CH2-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂- bestehenden Gruppe ausgewählt sind;
- R₅ aus der aus (C₂-C₂₀)Alkylen, (C₂-C₂₀)Alkenylen, Alkyloxy oder Oligoethylenglykol bestehenden Gruppe ausgewählt ist;
- R₆ aus bicyclischen Fragmenten von 1,4:3,6-Di-anhydrohexitolen der Strukturformel (II) 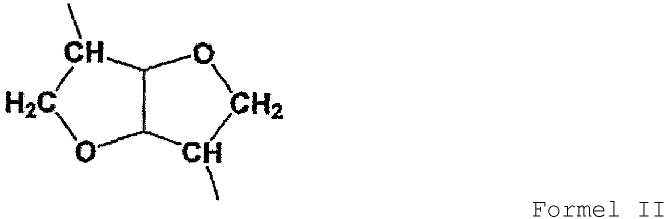 ausgewählt ist;
- R₇ für Wasserstoff, (C₆-C₁₀)Aryl, (C₁-C₆)Alkyl oder eine Schutzgruppe wie Benzyl- oder einen bioaktiven Stoff steht;
- R₈ unabhängig für (C₁-C₂₀)Alkyl oder (C₂-C₂₀)Alkenyl steht;
- R₉ oder R₁₀ unabhängig aus C₂-C₁₂-Alkylen oder C₂-C₁₂-Alkenylen ausgewählt sind,
- R₁₁ oder R₁₂ unabhängig aus H, Methyl, C₂-C₁₂-Alkylen oder C₂-C₁₂-Alkenylen ausgewählt sind.

9. Verfahren nach einem der Ansprüche 6-7, wobei das Polyesteramid (PEA) eine chemische Formel gemäß der Formel (III) aufweist, 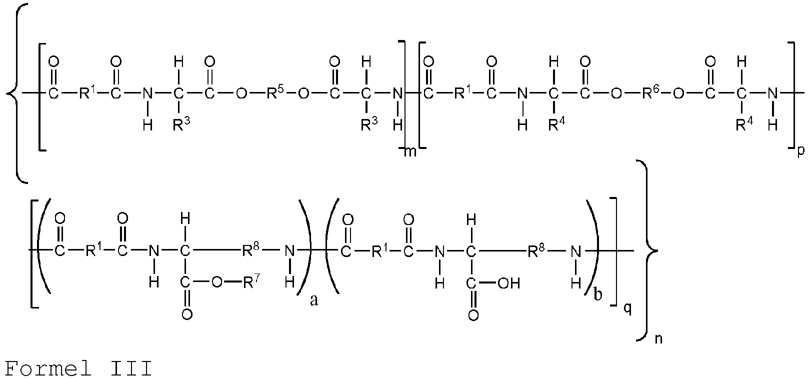 wobei
- m+p von 0,9-0,1 und q von 0,1 bis 0,9 variiert,
- m+p+q=1, wobei m oder p gleich 0 sein könnten, a wenigstens gleich 0,05 ist, b wenigstens gleich 0,05 ist, mit a+b=1,
- n gleich 5 bis 300 ist;
- R₁ unabhängig aus der aus (C₂-C₂₀)Alkylen, (C₂-C₂₀)Alkenylen, -(R₉-CO-O-R₁₀-O-CO-R₉)-, -CHR₁₁-O-CO-R₁₂-COOCR₁₁- und Kombinationen davon bestehenden Gruppe ausgewählt ist;
- R₃ und R₄ in einer einzelnen Grundgerüsteinheit m bzw. p unabhängig aus der aus Wasserstoff, (C₁-C₆)Alkyl, (C₂-C₆)Alkenyl, (C₂-C₆)-Alkinyl, (C₆-C₁₀)Aryl, (C₁-C₆)Alkyl, -(CH₂)SH, -(CH₂)₂S(CH₃), -CH₂OH, -CH(OH)CH₃, -(CH₂)₄NH₃+, -(CH₂)₃NHC(=NH₂+)NH₂, -CH₂COOH, -(CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂- bestehenden Gruppe ausgewählt sind;
- R₅ aus der aus (C₂-C₂₀)Alkylen, (C₂-C₂₀)-Alkenylen, Alkyloxy oder Oligoethylenglykol bestehenden Gruppe ausgewählt ist;
- R₆ aus bicyclischen Fragmenten von 1,4:3,6-Di-anhydrohexitolen der Strukturformel (II) ausgewählt ist;
- R₇ aus der aus (C₆-C₁₀)-Aryl, (C₁-C₆)-Alkyl bestehenden Gruppe ausgewählt ist;
- R₈ für -(CH₂)₄- steht;
- R₉ oder R₁₀ unabhängig aus C₂-C₁₂-Alkylen oder C₂-C₁₂-Alkenylen ausgewählt sind,
- R₁₁ oder R₁₂ unabhängig aus H, Methyl, C₂-C₁₂-Alkylen oder C₂-C₁₂-Alkenylen ausgewählt sind.

10. Verfahren nach einem der Ansprüche 1-9, wobei der bioaktive Stoff aus der Gruppe kleinmolekularer Arzneistoffe oder Biopharmazeutika ausgewählt ist.

11. Verfahren nach Anspruch 10, wobei das Biopharmazeutikum aus der Gruppe der Proteine, Peptide oder Polynukleotide ausgewählt ist.

12. Verfahren nach Anspruch 1, wobei wenigstens eine der Schichten ein Elastomermaterial umfasst.

13. Verfahren nach Anspruch 12, wobei das Elastomermaterial aus der aus Siliciumkautschuk, Ethylen-Propylen-Elastomeren oder Polyurethanen bestehenden Gruppe ausgewählt ist.

14. Verfahren nach Anspruch 13, wobei der Siliciumkautschuk aus der aus einem medizinisch und/oder für den Kontakt mit Lebensmitteln unbedenklichen Silikonkautschuk bestehenden Gruppe ausgewählt ist.

15. Arzneistoffzuführungssystem, erhältlich mit dem Verfahren nach einem der Ansprüche 1-14.

16. Arzneistoffzuführungssystem nach Anspruch 15 zur Verwendung in der Ophthalmologie, Herz-Kreislauf-, Schmerztherapie, Muskel-Skelett-, Krebsbehandlung oder bei der Impfstoffzuführung.

## Revendications

1. Procédé de fabrication d'un système d'administration de médicament à base de polymère comprenant un agent bioactif dispersé par l'intermédiaire des étapes de procédé suivantes :
(a) dissolution d'un polymère dans un solvant organique
(b) mélange du polymère dissous avec l'agent bioactif
(c) stratification du mélange entre au moins deux feuilles de matériau polymère, de sorte qu'au moins une feuille soit perméable au solvant organique,
(d) décollement des deux feuilles de polymère pour produire un film.

2. Procédé selon la revendication 1 dans lequel le film a une épaisseur dans la plage de 1 micromètre à 1 mm.

3. Procédé selon l'une quelconque des revendications 1 à 2 dans lequel le film est transformé en système d'administration de médicament adapté pour implantation ou injection.

4. Procédé selon l'une quelconque des revendications 1 à 3 dans lequel le système d'administration de médicament comprend des fibres, des bâtonnets, des disques, des enrobages, des tubes, des films ou des films enroulés.

5. Procédé selon la revendication 1 dans lequel le polymère est un polymère biostable, bioérodable ou biodégradable.

6. Procédé selon la revendication 5 dans lequel le polymère biostable, bioérodable ou biodégradable peut être choisi dans le groupe constitué de polyesteramides, polythioesters, polyesteruréthanes, polyesterurées, PLLA, PLGA, polyesters, polyanhydrides, polycarbonates, polyuréthanes, poly(acides aminés), polypeptides, polyamides, polysaccharides, polyéthers, polysulfones, poly(méth)acrylates, polysiloxanes, polyoléfines.

7. Procédé selon la revendication 6 dans lequel le polymère biodégradable est choisi parmi un polyesteramide comprenant des acides aminés.

8. Procédé selon l'une quelconque des revendications 6 à 7 dans lequel le polyesteramide (PEA) a une formule chimique selon la formule (I), 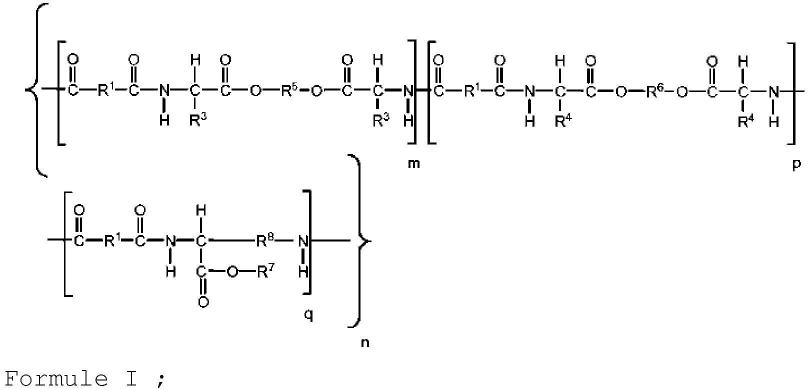 dans laquelle
- m varie de 0,01 à 0,99 ; p varie de 0 à 0,99 ; et q varie de 0,99 à 0,01 ; et dans laquelle n est de 5 à 100 ; et dans laquelle
- R₁ est indépendamment choisi dans le groupe constitué d'alkylène en C₂-C₂₀, alcénylène en C₂-C₂₀, -(R₉-CO-O-R₁₀-O-CO-R₉)-, -CHR₁₁-O-CO-R₁₂-COOCR₁₁ et des combinaisons de ceux-ci ;
- R₃ et R₄ dans un co-monomère unique m ou p, respectivement, sont indépendamment choisis dans le groupe constitué d'hydrogène, alkyle en C₁-C₆, alcényle en C₂-C₆, alcynyle en C₂-C₆, aryle en C₆-C₁₀, (alkyle en C₁-C₆, -(CH₂)SH, -(CH₂)₂S(CH₃), -CH₂OH, -CH(OH)CH₃, -(CH₂)₄NH₃+, -(CH₂)₃NHC(=NH₂+)NH2, -CH₂COOH, -(CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂-.
- R₅ est choisi dans le groupe constitué d'alkylène en C₂-C₂₀, alcénylène en C₂-C₂₀, alkyloxy ou oligoéthylèneglycol
- R₆ est choisi parmi des fragments bicycliques de 1,4:3,6-dianhydrohexitols de formule structurale (II) ; 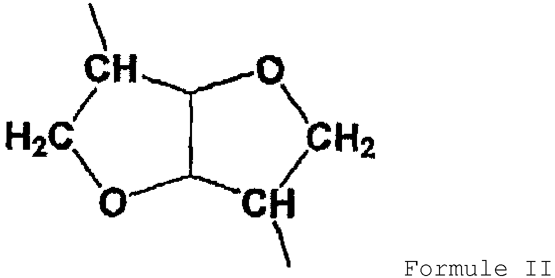
- R₇ est hydrogène, aryle en C₆-C₁₀, alkyle en C₁-C₆ ou un groupe protecteur tel que benzyl- ou un agent bioactif ;
- R₈ est indépendamment alkyle en C₁-C₂₀ ou alcényle en C₂-C₂₀ ;
- R₉ ou R₁₀ sont indépendamment choisis parmi alkylène en C₂-C₁₂ ou alcénylène en C₂-C₁₂ ;
- R₁₁ ou R₁₂ sont indépendamment choisis parmi H, méthyle, alkylène en C₂-C₁₂ ou alcénylène en C₂-C₁₂.

9. Procédé selon l'une quelconque des revendications 6 à 7 dans lequel le polyesteramide (PEA) a une formule chimique selon la formule (III), 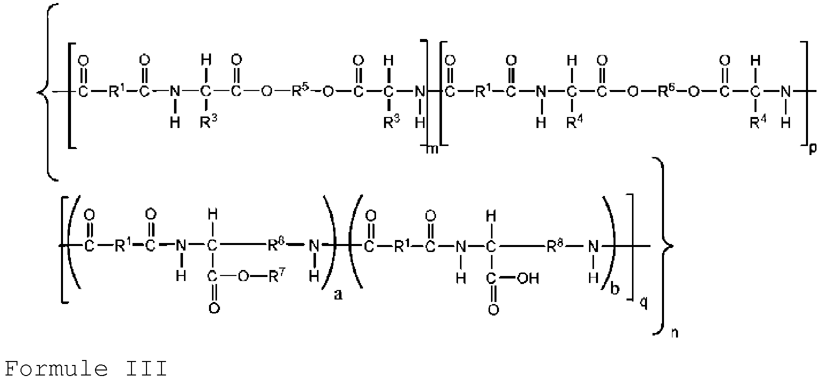 dans laquelle
- m+p varie de 0,9 à 0,1 et q varie de 0,1 à 0,9
- m+p+q=1 où m ou p peuvent être 0
a est au moins 0,05, b est au moins 0,05 où a+b=1
- n est 5 à 300 ;
- R₁ est indépendamment choisi dans le groupe constitué d'alkylène en C₂-C₂₀, alcénylène en C₂-C₂₀, -(R₉-CO-O-R₁₀-O-O-CO-R₉)-, -CHR₁₁-O-CO-R₁₂-COOCR₁₁- et des combinaisons de ceux-ci ;
- R₃ et R₄ dans un motif de squelette unique m ou p, respectivement, sont indépendamment choisis dans le groupe constitué d'hydrogène, alkyle en C₁-C₆, alcényle en C₂-C₆, alcynyle en C₂-C₆, aryle en C₆-C₁₀, alkyle en C₁-C₆, -(CH₂)SH, -(CH₂)₂S(CH₃), -CH₂OH, -CH(OH)CH₃, -(CH₂)₄NH₃⁺, -(CH₂)₃NHC(=NH₂+)NH2, -CH₂COOH, -(CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂-;
- R₅ est choisi dans le groupe constitué d'alkylène en C₂-C₂₀, alcénylène en C₂-C₂₀, alkyloxy ou oligoéthylèneglycol
- R₆ est choisi parmi des fragments bicycliques de 1,4:3,6-dianhydrohexitols de formule structurale (II)
- R₇ est choisi dans le groupe constitué de (aryle en C₆-C₁₀)-(alkyle en C₁-C₆)
- R₈ est -(CH₂)₄ ;
- R₉ ou R₁₀ sont indépendamment choisis parmi alkylène en C₂-C₁₂ ou alcénylène en C₂-C₁₂.
- R₁₁ ou R₁₂ sont indépendamment choisis parmi H, méthyle, alkylène en C₂-C₁₂ ou alcénylène en C₂-C₁₂.

10. Procédé selon l'une quelconque des revendications 1 à 9 dans lequel l'agent bioactif est choisi dans le groupe de médicaments à petite molécule ou d'agents biologiques.

11. Procédé selon la revendication 10 dans lequel l'agent biologique est choisi dans le groupe de protéines, peptides ou polynucléotides.

12. Procédé selon la revendication 1 dans lequel au moins une des feuilles comprend un matériau élastomère.

13. Procédé selon la revendication 12 dans lequel le matériau élastomère est choisi dans le groupe constitué de caoutchouc silicone, élastomères d'éthylène-propylène ou polyuréthanes.

14. Procédé selon la revendication 13 dans lequel le caoutchouc silicone est choisi dans le groupe constitué d'un caoutchouc silicone de qualité médicale et/ou de qualité pour contact alimentaire.

15. Système d'administration de médicament pouvant être obtenu par le procédé selon l'une quelconque des revendications 1 à 14.

16. Système d'administration de médicament selon la revendication 15 pour utilisation en ophtalmologie, médecine cardiovasculaire, prise en charge de la douleur, médecine musculo-squelettique, traitement du cancer ou dans l'administration de vaccin.
